# EUROPEAN PATENT SPECIFICATION

(11) **EP 4 497 399 B1**
(45) Date of publication and mention of the grant of the patent: **24.06.2026**
(21) Application number: 22819148.2
(22) Date of filing: 21.03.2022
(51) Int. Cl.: A61B 17/122, A61B 17/08, A61B 17/10, B23K 26/38, A61B 17/00

(54) **ONE-PIECE GRIPPING ELEMENT AND MANUFACTURING METHOD**
EINTEILIGES GREIFELEMENT UND HERSTELLUNGSVERFAHREN
ÉLÉMENT DE PRÉHENSION MONOBLOC ET PROCÉDÉ DE FABRICATION

(43) Date of publication of application: 29.01.2025
(73) Proprietor: Ningbo Xinwell Medical Technology Co., Ltd, Cixi, Zhejiang 315334 (CN)
(72) Inventor: HUANG, Junjun, Cixi, Zhejiang 315334 (CN); WU, Hailiang, Cixi, Zhejiang 315334 (CN); SHAN, Jian, Cixi, Zhejiang 315334 (CN); CHEN, Qingye, Cixi, Zhejiang 315334 (CN); SUN, Zhongli, Cixi, Zhejiang 315334 (CN)
(74) Representative: Murgitroyd & Company
(86) International application number: PCT/CN2022/082116
(87) International publication number: WO 2022/257543

(56) References cited:
- CN-A- 107 072 667
- CN-A- 107 920 813
- CN-U- 215 306 369
- JP-A- 2010 029 629
- US-A- 6 139 508
- US-A1- 2015 073 299
- US-A1- 2018 008 403
- US-A1- 2021 153 884
- US-A1- 2021 315 559

## Description

### TECHNICAL FIELD

The present disclosure relates to the field of medical devices, and in particular to a manufacturing method for a gripper in an insertable tissue clamp device.

### BACKGROUND

An insertable tissue clamp device is an insertable medical device configured to clamp tissues in a human body or an animal body for hemostasis or closure, including hemostatic clamps, tissue clamps, or the like.

For example, during minimally invasive treatment of digestive tract diseases, the tissue clamp device may generally be inserted through an instrument channel of an endoscope to achieve a purpose of treatment. For example, the hemostatic clamps (or the tissue clamps) have been widely used for hemostasis or closure at gastrointestinal bleeding or trauma sites.

In the prior art, a type of hemostatic clamps (or tissue clamps) mainly realizes opening and clamping through coordination of gripping arms and a sleeve. Specifically, left and right gripping arms are loosely assembled together by a pin, and when a gripping arm assembly is pulled toward the proximal side, the gripping arms thereof gradually retract into the sleeve and engage with a front edge of the sleeve. Limited by an outer diameter of the sleeve, the sleeve exerts a reverse compressing force on the gripping arms, and the gripping arms are elastically deformed inwards, and are thus closed. When the gripping arm assembly moves toward the distal side, the gripping arms are pushed out of the sleeve, and the gripping arms automatically reopen due to an elastic restoring force thereof, so that this gripping device can be opened and closed repeatedly.

In another type of hemostatic clamps (or tissue clamps), gripping arms thereof are connected mainly by a rotating shaft, and then the sleeve is provided with a rail for sliding up and down. The shaft may slide along the rail. A fixed shaft is disposed at the upper end of the sleeve. Elongated holes are disposed on the gripping arms. The fixed shaft extends through the elongated holes in the gripping arms. The two gripping arms are driven to move up and down by pushing and pulling the shaft to slide. After being blocked by the fixed shaft, the gripping arms are forced to move along paths in the elongated holes, thereby realizing opening and closing.

In the above structures, a gripping structure is formed by assembling a plurality of parts, which is not only troublesome to manufacture, but also requires precise assembling, resulting in a complex structural process.

US 6 139 508 A describes an articulated mechanism useful in devices such as forceps, single or multiple sample biopsy devices, articulated electrodes and the like.

US 2015/073299 A1 refers to soft tissue coring biospy devices and methods.

US 2018/008403 A1 relates to devices and methods of treating heart valve insufficiency.

US 2021/153884 A1 relates to devices and methods for removing acute blockages from blood vessels during intravascular medical treatments

US 2021/315559 A1 refers to manipulating medical devices, and particularly to a tube with a deformable portion connected to a manipulating portion.

### SUMMARY

The present invention is defined by the features of the independent claims. Embodiments of the invention are defined in the dependent claims.

The present disclosure is to mainly provide a manufacturing method for an integrated gripper, and an integrated gripper manufactured by the manufacturing method, which can reduce the number of components and parts and reduce assembling steps.

Based on the above objects, in an embodiment of the present disclosure, a manufacturing method for an integrated gripper is provided, including:
providing a tubular base material;
forming a plurality of gripping heads facing each other by cutting the base material through a laser;
forming a plurality of columns of first shrinkage slits distributed in a first direction of the base material by cutting the base material through the laser, the first shrinkage slits all extending in a second direction of the base material, and each column of the first shrinkage slits corresponding to one of the gripping heads in the first direction;
forming at least two gripping arms having first ends connected to each other and second ends separated from each other by cutting the base material in the first direction through the laser, each of the gripping arms including one column of the first shrinkage slits and one of the gripping heads; and
bending the gripping heads inwards to form a clamping jaw structure.

Based on the above objects, in an embodiment of the present disclosure, a manufacturing method for an integrated gripper is provided, including:
providing a sheet-like base material;
forming a plurality of gripping heads by cutting the base material through a laser;
forming a plurality of columns of first shrinkage slits distributed in a first direction of the base material by cutting the base material through the laser, the first shrinkage slits all extending in a second direction of the base material, and each column of the first shrinkage slits corresponding to one of the gripping heads in the first direction;
forming at least two gripping arms having first ends connected to each other and second ends separated from each other by cutting the base material in the first direction through the lase, each of the gripping arms including one of the first shrinkage slits and one of the gripping heads;
bending the base material around the first direction and fixing the bent base material to have a tubular shape; and
bending the gripping heads inwards to form a clamping jaw structure.

In an embodiment, the manufacturing method further includes:
forming a plurality of second shrinkage slits by cutting the base material in the second direction at side end surfaces on two sides of each column of the first shrinkage slits. The second shrinkage slits on a same side are arranged in the first direction. Two ends of each of the first shrinkage slits extend between two adjacent second shrinkage slits in the first direction. An overlapping region between the first shrinkage slits and the second shrinkage slits forms a twist deformable section, so that the bendable portion is capable of bending and twist deformation.

In an embodiment, a plurality of groups of first limiting blocks and second limiting blocks distributed in the first direction of the base material are formed by cutting for the second shrinkage slits. The first limiting blocks and the second limiting blocks face each other. The first limiting blocks and the second limiting blocks have first ends separated from each other and second ends connected into an entirety. Gaps arranged in the first direction are respectively defined between the first limiting blocks and the second limiting blocks. The second shrinkage slits are in communication with the gaps.

In an embodiment, each of the second shrinkage slits has a clearance in the first direction.

In an embodiment, each of the first limiting blocks and the second limiting blocks has a hook structure.

In an embodiment, the manufacturing method includes:
forming a notch extending in the first direction of the base material by cutting opposed ends of the gripping arms through the laser, to form an avoidance structure.

In an embodiment, the manufacturing method includes:
forming an engaging slot by cutting the first ends connected to each other of the gripping arms through the laser, the engaging slot being configured to lock the gripping arm.

In an embodiment, at least two engaging slots are provided and are distributed in the second direction of the base material.

In an embodiment, the manufacturing method includes:
forming a separation base by cutting an end of the base material facing away from the gripping arms through the laser, the separation base and the gripping arm being connected into an entirety through a first tearing portion.

In an embodiment, at least two first tearing portions are provided and are distributed in the second direction of the base material.

In an embodiment, the manufacturing method includes:
forming separation grooves recessed towards the gripping arms by cutting two sides of the first tearing portion through the laser, the first tearing portion being located at a recess.

In an embodiment, the manufacturing method includes:
forming a suspended suspension portion and cantilevers located on two sides of the suspension portion by cutting the separation base through the laser, the suspension portion being aligned with the first tearing portion.

In an embodiment, the manufacturing method includes:
forming a guide groove arranged in a first direction of the separation base by cutting a side of the suspension portion away from the first tearing portion through the laser, the suspension portion being disposed in the guide groove.

In an embodiment, the manufacturing method includes:
forming a stopper by cutting the separation base through the laser; and
pressing and deforming the stopper towards an interior of the separation base, to cause the stopper to protrude towards the interior of the separation base.

Based on the above objects, in an embodiment of the present disclosure, an integrated gripper is provided, which is manufactured by the manufacturing method as described in any one of the above embodiments.

In the manufacturing method for an integrated gripper according to the above embodiments, the method includes forming the integrated gripper with at least two gripping arms by cutting the tubular base material through the laser. The gripping arms each have a gripping head and first shrinkage slits distributed in the axial direction of the base material. With the first shrinkage slits, the bendable portion has a deformable structure capable of bending in a closing direction of the gripper and/or bending in an opening direction of the gripper. In this structure, a sleeve in an existing structure is omitted, with the deformation state of the bendable portion, the gripping arms can be opened and closed. When the gripper manufactured integrally is adopted, the entire gripping structure has fewer components and parts, a simpler structure, lower assembly requirements, and a greatly reduced cost.

### BRIEF DESCRIPTION OF THE DRAWINGS

FIG. 1 is a schematic structural view of a gripper (without a separation base) in a gripping state according to an embodiment of the present disclosure.
FIG. 2 is a schematic structural view of the gripper shown in FIG. 1 is in an opened state.
FIG. 3 is a schematic structural view of a gripper (with a separation base) in a gripping state according to an embodiment of the present disclosure.
FIG. 4 is a schematic structural view of the gripper shown in FIG. 3 is in an opened state.
FIG. 5 is a schematic view of a gripper in an unfolded shape according to an embodiment of the present disclosure, which may also be regarded as a schematic view showing a cutting when a sheet-like base material is machined to form the integrated gripper.
FIG. 6 is a schematic enlarged view of an unfolded deformable structure of a bendable portion in the embodiment shown in FIG. 5.
FIG. 7 is a schematic enlarged view of an unfolded deformable structure of a bendable portion according to another embodiment of the present disclosure, which may also be regarded as a schematic view showing a cutting when a sheet-like base material is machined to form the bendable portion.
FIG. 8 is a schematic enlarged view of an unfolded deformable structure of a bendable portion according to another embodiment of the present disclosure, which may also be regarded as a schematic view showing a cutting when a sheet-like base material is machined to form the bendable portion.
FIG. 9 is a schematic structural view of an insertable tissue clamp device according to an embodiment of the present disclosure, with a transmission assembly is partially omitted.
FIG. 10 is a partial sectional view of a connecting structure between the gripper and the transmission assembly according to an embodiment of the present disclosure.
FIG. 11 is a sectional view of the insertable tissue clamp device in an opened state (in which a moving rod moves in a first stroke) according to an embodiment of the present disclosure.
FIG. 12 is a schematic partial sectional view of the gripper in the state shown in FIG. 11.
FIG. 13 is a sectional view of the insertable tissue clamp device in a gripping state (in which the moving rod moves in a second stroke) according to an embodiment of the present disclosure.
FIG. 14 is a sectional view of the insertable tissue clamp device in the gripping state according to an embodiment of the present disclosure, with gripping arms locked by a locking structure (in which the moving rod moves in a third stroke).
FIG. 15 is a sectional view of the insertable tissue clamp device in the gripping state according to an embodiment of the present disclosure, with a retaining section and a separation section on the moving rod broken from a second tearing portion (in which the moving rod moves in the third stroke).
FIG. 16 is a sectional view of the insertable tissue clamp device in the gripping state according to an embodiment of the present disclosure, with the gripper and the separation base broken from a first tearing portion (in which the moving rod moves in the third stroke).
FIG. 17 is a schematic structural view of the deformable structure of the bendable portion when the gripper grips thinner tissue according to an embodiment of the present disclosure.
FIG. 18 is a schematic structural view of the deformable structure of the bendable portion when the gripper grips thicker tissue according to an embodiment of the present disclosure.
FIG. 19 is a schematic structural view of the gripping arm and the separation base that are being broken through the first tearing portion according to an embodiment of the present disclosure.
FIG. 20 is a schematic structural view of the first tearing portion in an unbroken state according to an embodiment of the present disclosure.
FIG. 21 is a schematic structural view of the first tearing portion in a broken state according to an embodiment of the present disclosure.

### DETAILED DESCRIPTION

The present disclosure will be described in detail below through specific embodiments with reference to the drawings. Similar elements in different embodiments are designated by similar reference numbers. In the following embodiments, many details are set forth so that the present disclosure can be better understood. However, those skilled in the art can easily recognize that some of the features can be omitted in different situations, or can be replaced by other elements, materials, or methods. In some cases, some operations related to the present disclosure are not shown or described in the specification so as to avoid the core part of the present disclosure being overwhelmed by too many descriptions. For those skilled in the art, the detailed description of these operations is not necessary, and these operations can be fully understood according to the description in the specification and the common technical knowledge in the art.

In addition, the characteristics, operations or features described in the specification may be combined in any appropriate manner to form various embodiments. Moreover, the steps or actions in the described methods may also be changed or adjusted in the order in a manner obvious to those skilled in the art. Therefore, the orders in the specification and the drawings are only for clearly describing a certain embodiment, but not mean a necessary order, unless otherwise stated that a certain order must be followed.

Herein, serial numbers, such as "first" and "second", are used to distinguish the described objects, but do not have any order limitation or technical meaning. The "connection" and "coupling" mentioned in the present disclosure, unless otherwise specified, may include direct and indirect connection (coupling).

In this embodiment, a plurality of manufacturing methods for an integrated gripper in an insertable tissue clamp device (for convenience of description, hereinafter referred to as a clamp device) are provided. The clamp device is configured to clamp tissue in a human or animal body (collectively referred to as targets) for hemostasis or closure, which may include, but is not limited to, hemostatic clamps, tissue clamps, or the like.

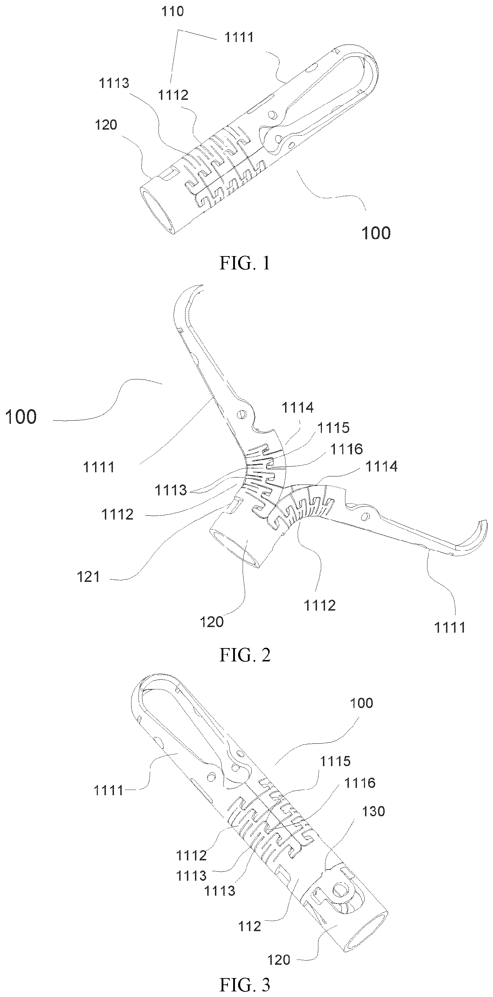

Referring to FIGS. 1 and 2, in an embodiment, an integrated gripper 100 is manufactured by taking a tubular base material as a machining basis. The integrated gripper 100 is not limited to a structure shown in FIGS. 1 and 2.

The manufacturing method includes the following steps.

At S11, a tubular base material is provided.

In the step, the tubular material may be a metal tube of 0.25 mm to 0.15 mm. Certainly, other tubes with other thicknesses and materials are also available.

At S12, a plurality of gripping heads 1111 facing each other are formed by cutting the base material through a laser.

Two or more gripping heads 1111 may be provided.

At S13, a plurality of columns of first shrinkage slits 1113 distributed in a first direction of the base material are formed by cutting the base material through a laser. The first shrinkage slits 1113 all extend in a second direction of the base material. Each column of the first shrinkage slits 1113 corresponds to one of the gripping heads 1111 in the first direction.

In this step, each column of the first shrinkage slits 1113 may form a bendable portion 1112. By use of the bendable portion 1112, the gripping head 1111 can bend towards one or more sides, to realize opening and closing of the gripper 100.

When a tubular material is used as the base material, the first direction is an axial direction of the base material. The second direction is a circumferential direction of the base material.

At S14, at least two gripping arms 111 having first ends connected to each other and second ends separated from each other are formed by cutting the base material in the first direction through the laser. Each of the gripping arms 111 includes one column of the first shrinkage slits 1113 and one of the gripping heads 1111.

Each gripping arm 111 is a movable unit. Through the combination of different gripping arms 111, an opened state and a gripping state of the entire gripper 100 can be realized.

At S15, the gripping heads 1111 are bent inwards to form a clamping jaw structure.

In this step, after the gripping heads 1111 extending in the first direction of the base material are bent, the clamping jaw structure as shown in FIGS. 1 and 2 may be formed.

The steps shown in this embodiment may be performed in any feasible order and are not limited to the order as described above. In particular, in the case of laser cutting, a corresponding part may be flexibly selected for cutting according to a requirement, which is not limited to performing cutting in the above order. For example, orders of cutting actions for the gripping heads 1111, the first shrinkage slits 1113, the gripping arms 111, and other features as described below may be combined in any way without causing any mutual conflict.

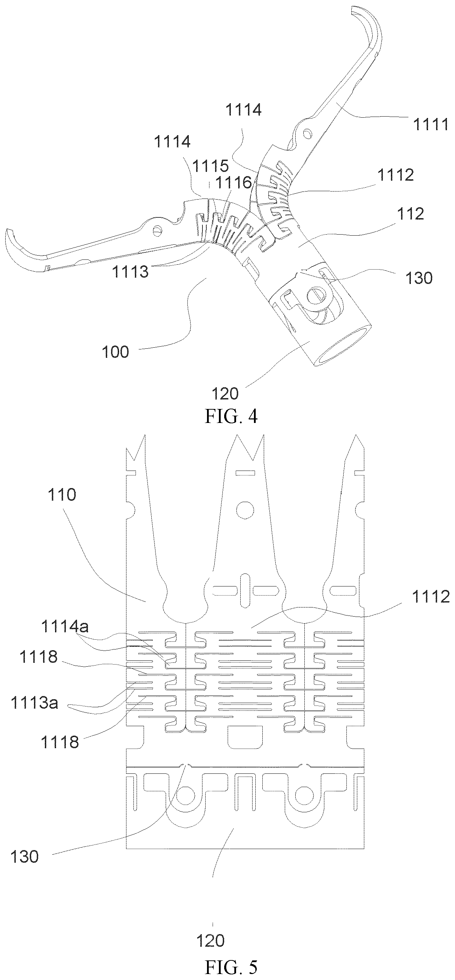

Referring to FIGS. 5 to 8, in an embodiment, a manufacturing method for manufacturing the integrated gripper 100 with a sheet is further provided. The method includes the following steps.

At S21, a sheet-like base material is provided.

In this step, the tubular material may be a metal sheet of 0.25 mm to 0.15 mm. Certainly, other sheets with other thicknesses and materials are also available.

At S22, a plurality of gripping heads 1111 are formed by cutting the base material through a laser.

In this step, after the gripping heads 1111 extending in the first direction of the base material are bent, the clamping jaw structure as shown in FIGS. 1 and 2 may be formed.

When a sheet is used as the base material, the first direction is a length direction of the base material, that is, an up-down direction in the figures shown in FIGS. 5 to 8. The second direction is a width direction of the base material, that is, a left-right direction in the figures shown in FIGS. 5 to 8.

At S23, a plurality of columns of first shrinkage slits 1113 distributed in the first direction of the base material are formed by cutting the base material through a laser. The first shrinkage slits 1113 all extend in the second direction of the base material. Each column of the first shrinkage slits 1113 corresponds to one of the gripping heads 1111 in the first direction.

At S24, at least two gripping arms 111 having first ends connected to each other and second ends separated from each other are formed by cutting the base material in the first direction through a laser. Each of the gripping arms 111 includes one column of the first shrinkage slits 1113 and one of the gripping heads 1111.

At S25, the base material is bent around the first direction and fixed to have a cylindrical shape.

After the sheet-like base material is made into a cylindrical structure, the gripper 100 as shown in FIGS. 1 and 2 may be obtained. For example, the structure shown in FIG. 5 is bent, and left and right sides thereof are fixed, and then the cylindrical structure shown in FIGS. 1 and 2 may be obtained. The fixing may be welding, bonding, mechanical connection (such as screwing or engaging), or the like.

At S26, the gripping heads 1111 are bent inwards to form a clamping jaw structure.

Similarly, the steps shown in this embodiment may be performed in any feasible order and are not limited to the order as described above. In particular, in the case of laser cutting, a corresponding part may be flexibly selected for cutting according to a requirement, which is not limited to performing cutting in the above order. For example, orders of cutting actions for the gripping heads 1111, the first shrinkage slits 1113, the gripping arms 111, and other features as described below may be combined in any way without causing any mutual conflict.

Further, in the two manufacturing methods as above, referring to FIGS. 1 to 8, the following step may also be included.

A plurality of second shrinkage slits 1118 are formed by cutting the base material in the second direction at side end surfaces on two sides of each column of the first shrinkage slits 1113 through a laser. The second shrinkage slits 1118 on the same side are arranged in the first direction. Two ends of each of the first shrinkage slits 1113 extend between two adjacent second shrinkage slits 1118 in the first direction. An overlapping region between the first shrinkage slits 1113 and the second shrinkage slits 1118 forms a twist deformable section 1119, so that the bendable portion 1112 is capable of bending and twist deformation.

Further, in the two manufacturing methods as above, referring to FIGS. 1 to 8, a plurality of groups of first limiting blocks 1115 and second limiting blocks 1116 distributed in the first direction of the base material are formed on at least one side of each column of first shrinkage slits 1113 by the cutting for the respective second shrinkage slits 1118. The first limiting blocks 1115 and the second limiting blocks 1116 face each other. The first limiting blocks 1115 and the second limiting blocks 1116 have first ends separated from each other and second ends connected into an entirety. Gaps 1117 arranged along the first direction are provided between the first limiting blocks 1115 and the second limiting blocks 1116 respectively. The second shrinkage slits 1118 are in communication with the gaps 1117, respectively.

In an embodiment, the second shrinkage slits 1118 are respectively provided with gaps in the first direction.

Referring to FIGS. 1 to 8, in an embodiment, each of the first limiting blocks 1115 and the second limiting blocks 1116 has a hook structure.

In the two manufacturing methods as above, referring to FIGS. 1 to 8, the following step may also be included.

A notch extending in the first direction of the base material is formed by cutting opposed ends of the gripping arms through a laser, to form an avoidance structure 1110.

In the two manufacturing methods as above, referring to FIGS. 1 to 8, the following step may also be included.

An engaging slot 1121 configured to lock the gripping arm 111 is formed by cutting the first ends that are connected to each other of the gripping arms 111 through a laser.

Referring to FIGS. 1 to 8, in an embodiment, at least two engaging slots are provided and are distributed in the second direction of the base material.

In the two manufacturing methods above, referring to FIGS. 3 to 5, the following step may also be included.

A separation base 120 is formed by cutting an end of the base material facing away from the gripping arms 111 through a laser. The separation base 120 and the gripping arms 111 are connected into an entirety through a first tearing portion 130.

Referring to FIGS. 3 to 5, in an embodiment, at least two first tearing portions 130 are provided and are distributed in the second direction of the base material.

In the two manufacturing methods above, referring to FIGS. 3 to 5, the following step may also be included.

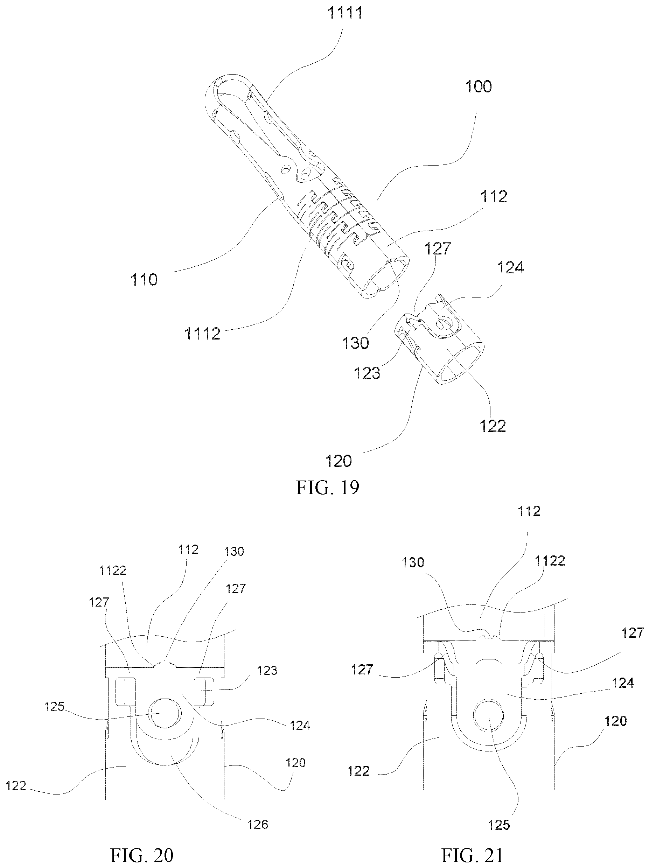

Separation grooves (denoted by 1122 shown in FIG. 20) recessed towards the gripping arms 111 are formed by cutting two sides of the first tearing portion 130 through a laser. The first tearing portion 130 is located at such recessed portion.

In the two manufacturing methods above, referring to FIGS. 3 to 5, the following step may also be included.

A suspended suspension portion 121 and cantilevers 127 located on two sides of the suspension portion 124 are formed by cutting the separation base through a laser. The suspension portion 124 is aligned with the first tearing portion 130.

In the two manufacturing methods above, referring to FIGS. 3 to 5, the following step may also be included.

A guide groove 126 extending along the first direction of the separation base 120 is formed by cutting a side of the suspension portion 124 away from the first tearing portion 120 through a laser. The suspension portion 124 is disposed in the guide groove 126.

In the two manufacturing methods above, referring to FIGS. 3 to 5, the following steps may also be included.

A stopper 121 is formed by cutting the separation base 120 through a laser.

The stopper 121 is pressed and deformed towards an interior of the separation base 120, to cause the stopper 121 to protrude towards the interior of the separation base 120.

On the other hand, the embodiment further provides a clamp device. An integrated gripper in the clamp device may be manufactured by the methods as illustrated in the above embodiments.

Referring to FIGS. 1 to 16, the clamp device includes a gripper 100, a moving rod 200, a transmission assembly 300, and a control handle 400.

In this embodiment, the gripper 100 are integrally formed. When the laser is used to performing cutting, extremely small gaps can be machined, which is beneficial to miniaturization of the overall structure and improvement of structural compactness.

Referring to FIGS. 1 to 4, the gripper 100 includes a gripping body 110 and a separation base 120. The gripping body 110 and the separation base 120 are connected into an entirety through the first tearing portion 130. The first tearing portion 130 allows an operator to separate the gripping body 110 from the separation base 120 through an external force.

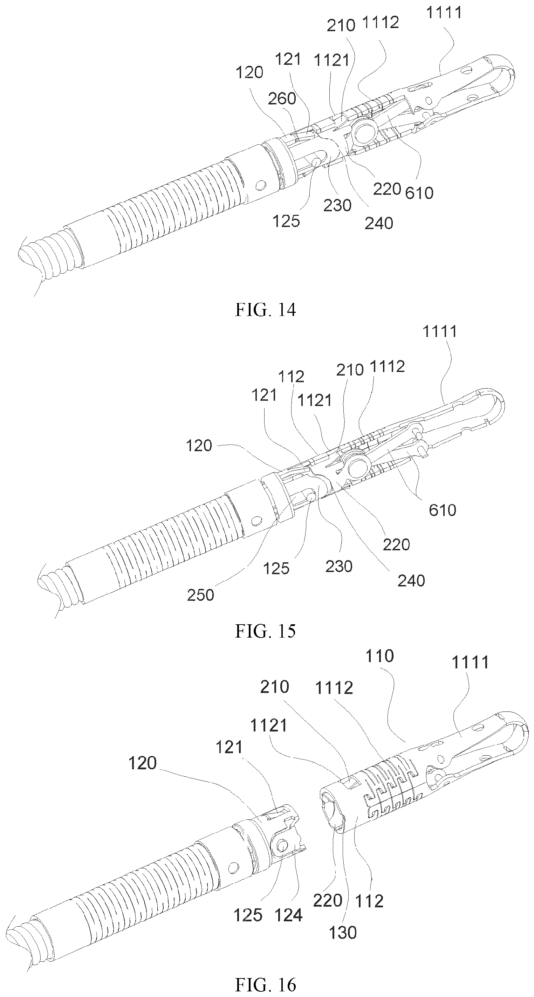

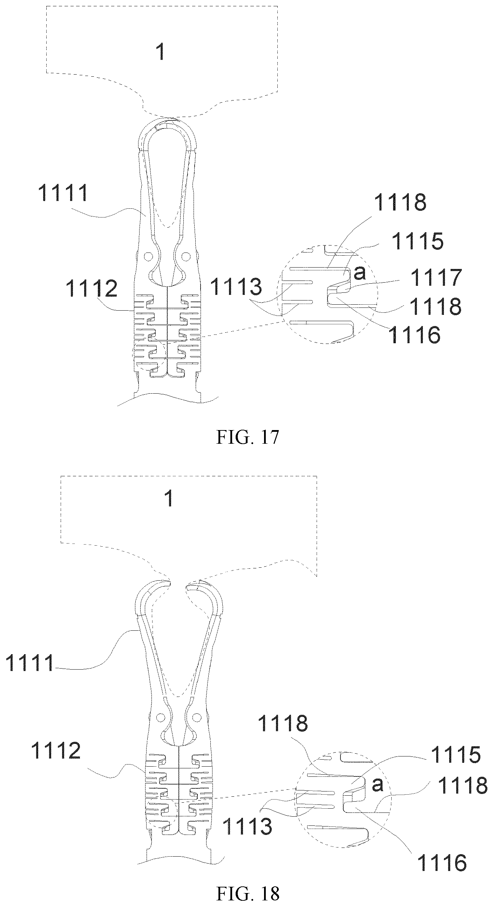

The gripping body 110 includes at least two gripping arms 111. The gripping arms 111 are connected into an entirety. Each gripping arm 111 includes a gripping head 1111 and a bendable portion 1112. The gripping arms 111 forms a clamping jaw structure to clamp a target. The clamping jaw structure is a structure that can firmly grasp the target. For example, in FIGS. 1 to 4, when two gripping arms 111 are provided, the two gripping arms 111 face each other. When the two gripping arms 111 are closed as shown in FIG. 1 (in the gripping state in this case), the target may be grasped, as shown in FIGS. 16 and 17.

Referring to FIGS. 1 to 4, in an embodiment, the bendable portion 1112 has a semicylindrical structure, a sheet shape, or other structures, such as a sheet shape. When the gripping body 110 is closed, the bendable portion 1112 may enclose a cylindrical structure.

Different from the prior art in which the gripping arms are opened and closed by limiting the gripping arms with a sleeve, in this embodiment, the opening and closing of the gripping arms 111 mainly rely on deformation of the bendable portion 1112. The bendable portion 1112 has a deformable structure capable of bending in a closing direction of the gripping body 110 and/or bending in an opening direction of the gripping body 110. Referring to FIGS. 1 and 3, in the illustrated embodiment, an initial state of the gripping body 110 is a gripping state. That is, the bendable portion 1112 is in a gripping state without deformation. In this case, the bendable portion 1112 has at least the deformable structure capable of bending in the direction opening the gripping body 110, so as to open the gripping body 110 as shown in FIGS. 2 and 4.

The gripping head 1111 has higher resistance to bending deformation than the bendable portion 1112 to ensure that the gripping arms 111 can provide a better grasping effect for the target. Bending deformation of the bendable portion 1112 is achieved through the integrated structure thereof. The bending deformation of the bendable portion 1112 is reversible. That is, the bendable portion 1112 has elasticity and can rebound and reset when the external force is removed, so the bending deformation can be repeated.

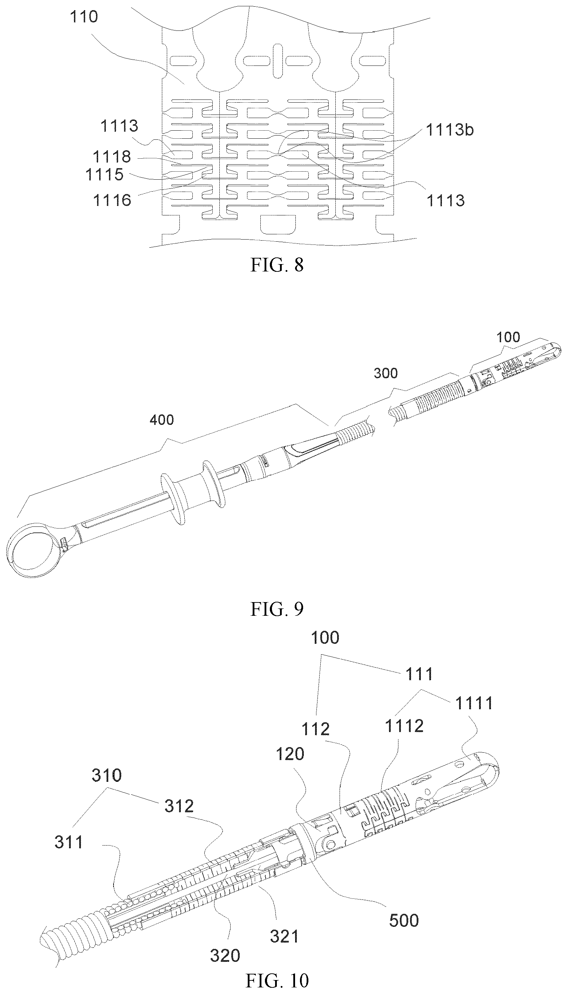

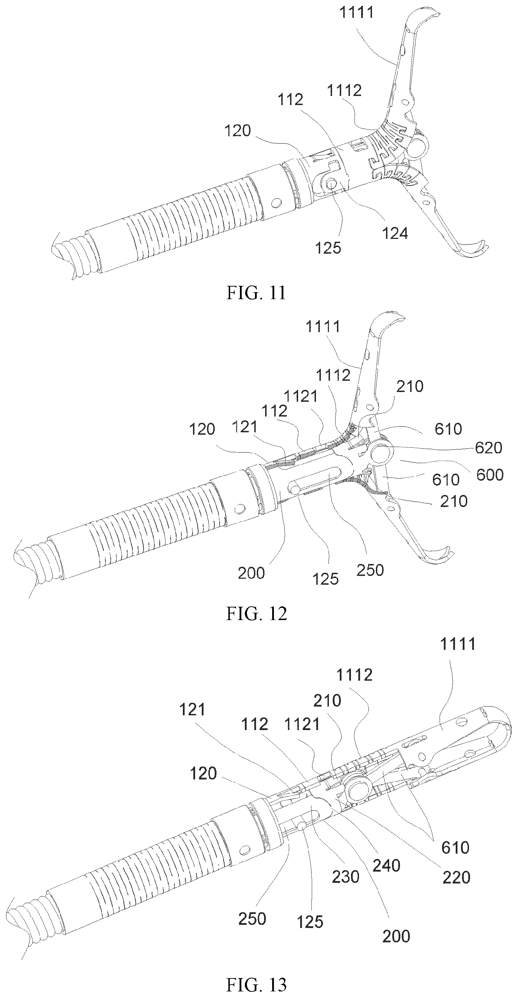

The moving rod 200 is configured to control opening and gripping states of the gripper 100. In FIGS. 11 to 16, the moving rod 200 is a pull rod. The moving rod 200 is connected to the gripping body 110. Motion of the moving rod 200 can control the gripping body 110 to move in the opening direction and in the gripping direction. The transmission assembly 300 plays roles in supporting the gripper 100 and transferring motion and force to the moving rod 200. Referring to FIGS. 9 and 10, the transmission assembly 300 includes a sleeve assembly 310 and a transmission member 320 extending into the sleeve assembly 310. The transmission member 320 is connected to the moving rod 200. The separation base 120 of the gripper 100 is rotatably connected to the sleeve assembly 310, for example, mounted on the sleeve assembly 310 through a rotating base 500, so that the gripper 100 can rotate as a whole relative to the sleeve assembly 310. The sleeve assembly 310 is connected to the control handle 400. The control handle 400 and the transmission member 320 form a linkage structure to control actions of the transmission member 320, the moving rod 200, and the gripper 100. For example, the operator may control rotation of the gripper 100 relative to the sleeve assembly 310 by the control handle 400, and may also control the opening and closing of the gripper 100 by the control handle 400.

The moving rod 200 has a first stroke, a second stroke, and a third stroke. The first stroke, the second stroke, and the third stroke are three parts of the entire moving stroke of the moving rod 200. The three strokes may be in a same direction, or at least two strokes may be in different directions.

As an example, referring to FIGS. 11 and 12, in this case, the moving rod 200 is in the first stroke, and when the moving rod 200 moves away from the control handle 400 along an axial direction thereof and moves approaching the gripping body 110 (i.e., moves to the right side in the figure), the moving rod 200 can drive the gripping body 110 to open outwards, thereby moving the gripping body 110 to the opened state.

Referring to FIG. 13, in this case, the moving rod 200 is in the second stroke, and when the moving rod 200 moves approaching to the control handle 400 along the axial direction thereof and moves away from the gripping body 110 (i.e., moves to the left side in the figure), the moving rod 200 can drive the gripping body 110 to approach each other inwards, thereby moving the gripping body 110 to the gripping state.

Referring to FIGS. 3 to 16, in this case, the moving rod 200 is in the third stroke, and when the moving rod 200 moves approaching the control handle 400 along the axial direction thereof and moves away from the gripping body 110 (i.e., moves to the left side in the figure), the third stroke and the second stroke are in a same direction and are closely connected. That is, after the gripping body 110 moves to the gripping state, the moving rod 200 switches from the second stroke to the third stroke. The third stroke may be divided into a plurality of sub-strokes. The sub-strokes include a locking stroke, an inner disengagement stroke, and an outer disengagement stroke.

Referring to FIG. 14, when the moving rod 200 switches to the third stroke until it moves to the position shown in the figure, the gripping body 110 is locked in this case, so that the moving rod 200 cannot move in a reverse direction and open the gripping body 110 again. In this process, the moving stroke of the moving rod 200 is the locking stroke.

Referring to FIG. 15, after finishing the locking stroke, the moving rod 200 moves into the inner disengagement stroke. When the moving rod 200 moves to the position shown in the figure, the gripping body 110 is separated from the moving rod 200, the moving rod 200 does not drive the gripping body 110 to move and lose the control over the gripping body 110, so that the gripping body 110 is kept in a locked state. In this process, the moving stroke of the moving rod 200 is the inner disengagement stroke.

Referring to FIG. 16, after finishing the inner disengagement stroke, the moving rod 200 moves into the outer disengagement stroke. When the moving rod 200 moves to the position shown in the figure, the gripping body 110 and the separation base 120 are broken from the first tearing portion 130. At this point, the gripping body 110 is left on the target clamped. The separation base 120, the moving rod 200, and the transmission assembly 300 may be withdrawn from the body of the target. In this process, the moving stroke of the moving rod 200 is the outer disengagement stroke.

In the structures shown in the above embodiments, the sleeve in the existing structure is omitted, and instead, the moving rod 200 directly drives the gripping body 110, with a deformable state of the bendable portion 1112, the gripping body 110 can be closed or opened. Since the sleeve has a limiting effect on the gripping arm 111 and the sleeve is omitted, the gripping body 110 starts to deform from the bendable portion 1112, and a deformation region of the gripping body 110 is closer to the bottom of the entire gripping body 110. Therefore, under a same opened width requirement, a length of the integrated gripping body 110 is shorter than a length of a structure combining the gripping arm 111 and the sleeve in the prior art. Under the same length, the integrated gripping body 110 can open to a larger angle than the structure combining the gripping arm 111 and the sleeve in the prior art, making it easier to grasp the tissue of the target. After the gripping body 110 is separated from the separation base 120, this shorter gripping body 110 can temporarily stay in the target, which can reduce discomfort caused by an excessively long gripping head left by a hemostatic clamp (or tissue clamp), and can minimize the problem of excessive wear and tear on the target due to the excessively long gripping head left by the hemostatic clamp (or tissue clamp). In addition, the integrated structure prevents fit clearance of components and parts required for shaft hole fit or sliding displacement, so the gripping arm 111 has higher bending repetition accuracy.

Moreover, in addition to the integrated manufacturing of the gripping body 110, in this embodiment, the separation base 120 required to be separated from the body of a surgical subject and the gripping body 110 are also integrally manufactured. The entire gripper 100 has a simple machining process. Compared with a combined structure of a plurality of components and parts in the existing hemostatic clamp (or tissue clamp), after the use of the gripper 100 manufactured integrally, the entire clamping structure has fewer parts, a simpler structure, lower assembly requirements, a greatly reduced cost, and higher control accuracy. Similarly, the length of the entire gripper 100 is shorter than that of the existing hemostatic clamp (or tissue clamp). Since an inner diameter of an endoscopic instrument channel is very limited, it is easier for this shorter gripper 100 to extend through the endoscopic instrument channel.

Further, as described above, the bending deformation of the bendable portion 1112 is achieved through an integrated structure thereof. Referring to FIGS. 1 to 8, in some embodiments, an end of the gripping body 110 adjacent to the separation base 120 is a proximal end, and another end away of the gripping body 110 from the separation base 120 is a distal end. A direction from the proximal end of the gripping body 110 to the distal end thereof is a first direction of the gripping body 110. In order to realize the integrated deformable structure, the deformable structure includes a plurality of first shrinkage slits 1113. The first shrinkage slits 1113 are arranged in sequence in the first direction.

In an embodiment, as shown in FIGS. 1 and 3, the gripping body 110 in the initial state is maintained in the gripping state, the first shrinkage slits 1113 are maintained in the initial state, and each part of the bendable portion 1112 does not deform. As shown in FIGS. 2 and 4, when the gripping body 110 is required to open, the bendable portion 1112 deforms outwards, and the first shrinkage slit 1113 shrinks and deforms, thereby shrinking the outside of the bendable portion 1112 (the sides of the gripping arms 111 away from each other), so that the whole gripping head 1111 opens.

Referring to FIGS. 1 to 4, in an embodiment, the first shrinkage slit 1113 extends in a circumferential direction of the bendable portion 1112. The first shrinkage slits 1113 are arranged parallel to each other. Certainly, in addition to being parallel to each other, the first shrinkage slits 1113 may also be arranged in other non-parallel manners. The first shrinkage slits 1113 are all arranged in the circumferential direction of the bendable portion 1112 in parallel, which can unify bending deformation directions of the first shrinkage slits 1113, so that the bending deformation of the gripping body 110 is smoother and more stable.

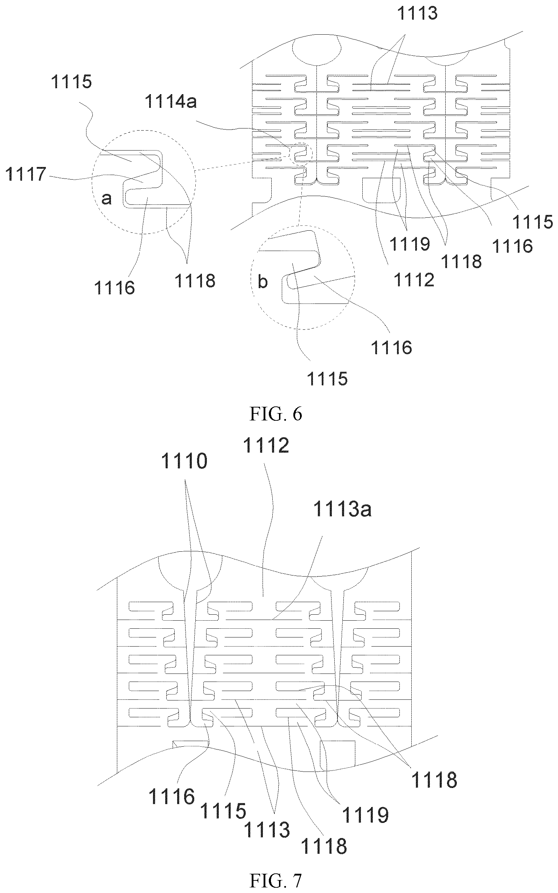

To achieve smoother bending changes, in an embodiment, the first shrinkage slits 1113 are divided into five groups, and each group of first shrinkage slits 1113a includes at least one first shrinkage slit 1113. As shown in FIGS. 1 to 6, in this embodiment, each group of first shrinkage slits 1113a includes two first shrinkage slits 1113. As shown in FIGS. 7 and 8, in this embodiment, each group of first shrinkage slits 1113a includes one first shrinkage slit 1113. Shrinkage of each first shrinkage slit 1113 enables the bendable portion 1112 to have a certain bending angle. In combination with a plurality of groups of first shrinkage slits 1113, the bendable portion 1112 may have a larger opening/closing angle. A length of a combination of all the first shrinkage slits 1113 in the first direction determines a bending deformation region of the whole bendable portion 1112. The number of the groups of first shrinkage slits 1113a, the gap between two adjacent first shrinkage slit group 1113a in the first direction, and the number of the first shrinkage slits 1113 in the group of first shrinkage slits 1113a may be flexibly set as required. For example, 4 to 6 groups of first shrinkage slits 1113a may be provided.

Referring to FIG. 8, in an embodiment, the first shrinkage slit 1113 is in the shape of an elongated groove. A middle part of the first shrinkage slit 1113 has two arc-shaped edges 1113b protruding relative to each other. When the gripper 100 is opened to a determined position, the arc-shaped edges 1113b may contact each other, thereby determining a maximum opening angle. When the gripper 100 is in the gripping state, the arc-shaped edges 1113b may contact each other, thereby providing a support force for the gripper 100.

In consideration of a requirement for minimally invasive surgery, the clamp device generally has a very delicate and compact structure. Therefore, on the premise of meeting a small volume of the clamp device, it is generally inappropriate that the gripper 100 is made of a material with a larger thickness. However, a thinner thickness requirement may result in weakening of strength of the bendable portion 1112. Specifically, as shown in FIG. 4, when the external force exerted by the operator is excessively large such that the gripping arms 111 are bent outwards at an excessive angle, the gripping arms 111 may be broken at the bendable portion 1112.

Based on this, in an embodiment, as shown in FIG. 4, the bendable portion 1112 has limiting structures 1114. The limiting structures 1114 are configured to limit a maximum angle at which the bendable portion 1112 is bent in the opening direction.

Referring to FIGS. 1 to 8, in an embodiment, each limiting structure 1114 includes a plurality of limiting elements 1114a arranged in the first direction of the gripping body 110. The limiting element 1114a includes a first limiting block 1115 and a second limiting block 1116 that face each other. As shown in two partial enlarged views in FIG. 6a and 6b, a gap 1117 arranged in the first direction is provided between the first limiting block 1115 and the second limiting block 1116. In the initial state, the gap 1117 is formed between the first limiting block 1115 and the second limiting block 1116 as shown in FIG. 6a. When the gripping body 110 gradually opens outwards, the first limiting block 1115 and the second limiting block 1116 move relative to each other in the first direction, and the gap 1117 gradually becomes smaller. Finally, when the bendable portion 1112 reaches the maximum angle, as shown in FIG. 6b, the first limiting block 1115 and the second limiting block 1116 fit with each other to form position limiting.

Referring to FIGS. 5 to 8, in an embodiment, the first limiting block 1115 and the second limiting block 1116 are two limiting hook structures that interlock with each other. The limiting hook structure may alternatively be replaced with other structures with similar functions. Referring to FIGS. 1 to 4, in an embodiment, the first shrinkage slit 1113 is located in the middle of the bendable portion 1112 in the circumferential direction thereof. At least two groups of limiting structures 1114 are provided. In the circumferential direction of the bendable portion 1112, the bendable portion 1112 is provided with the limiting structures 1114 on two sides respectively, which can further ensure that the entire bendable portion 1112 can synchronize bending changes and position limiting.

Referring to FIGS. 1 to 8, in an embodiment, in the same limiting element 1114a, the first limiting block 1115 and the second limiting block 1116 are formed by cutting in the sidewalls of the bendable portion 1112 located on sides of the first shrinkage slit 1113. First ends of the first limiting block 1115 and the second limiting block 1116 adjacent to the first shrinkage slit 1113 are connected into an entirety, and second ends of the first limiting block 1115 and the second limiting block 1116 are separated from each other. When the gripping body 110 opens outwards, the first limiting block 1115 and the second limiting block 1116 may also open along with the gripping body 110.

As shown in FIGS. 1 to 8, each group of first shrinkage slits 1113a may be aligned with one limiting element 1114a in the circumferential direction, thereby ensuring that a limiting effect of the limiting element 1114a can accurately act on the corresponding first shrinkage slit 1113, to prevent breakage of the bendable portion 1112 caused by continuous shrinkage and deformation of the first shrinkage slit 1113 after the bendable portion bends to the maximum angle.

The number of the limiting elements 1114a may be greater than that of the groups of first shrinkage slits 1113a, thereby completely covering all the first shrinkage slits 1113 in the first direction, to achieve a better limiting effect. Certainly, the number of the limiting element 1114a may alternatively be less than or equal to that of groups of first shrinkage slits 1113a.

Further, referring to FIGS. 5 to 8, in an embodiment, second shrinkage slits 1118 at least partially arranged along the circumferential direction of the bendable portion 1112 are provided between the first limiting block 1115 and the second limiting block 1116. The second shrinkage slit 1118 can separate the first limiting block 1115 from the second limiting block 1116 so that the first limiting block 1115 and the second limiting block 1116 can move relative to each other. The second shrinkage slit 1118 is in communication with the gap 1117 between the first limiting block 1115 and the second limiting block 1116.

Bending motion in the opening direction and bending motion in the gripping direction of the gripping body 110 are often accompanied by twist motion around the circumferential direction thereof. Therefore, referring to FIGS. 5 to 8, in an embodiment, two ends of each group of first shrinkage slits 1113a respectively extend between two adjacent second shrinkage slits 1118 of the limiting element 1114a in the first direction. An overlapping region between the first shrinkage slit 1113 and the second shrinkage slit 1118 forms a twist deformable section 1119, so that the bendable portion 1112 is capable of bending and twist deformation. When the twist deformable section 1119 is provided, the bending deformation of the gripping body 110 is smoother, and breakage of the bendable portion 1112 due to a twisting force is also prevented. By adjusting a circumferential length and a longitudinal height of the twist deformable section 1119, a maximum opening angle, bending softness or support capability of the bendable portion 1112 can be further changed, which may be flexibly set according to actual requirements.

In the embodiments shown in FIGS. 6 and 8, the second shrinkage slit 1118 is shaped as a straight line. In the embodiment shown in FIG. 7, the second shrinkage slit 1118 is U-shaped.

Further, referring to FIGS. 17 and 18, the second shrinkage slit 1118 has a clearance in the first direction. The second shrinkage slits 1118 of the limiting structure 1114 form an adaptive floating structure that can be deformed in the gripping direction.

Referring to FIG. 17, when the gripping body 110 grips a thinner target 1, the gripping body 110 may be closed normally, and the second shrinkage slit 1118 maintains a normal clearance (as shown in the partial enlarged view in FIG. 17a). Referring to FIG. 18, when the gripping body 110 grips a thicker target 1, the gripping body 110 may not be closed to the extent shown in FIG. 17. In this case, the moving rod 200 is continuously pulled, and the second shrinkage slit 1118 may be deformed in the closing direction of the gripping body 110 (as shown in the partial enlarged view in FIG. 18a). For example, in an embodiment, each second shrinkage slit 1118 can provide a clearance in the range of 0.02 mm to 0.05 mm in the first direction, that is, have a compression amount of 0.02 mm to 0.05 mm, so that the bendable portion 1112 may be bent and deformed inwards as shown in FIG. 18 (in FIG. 18, two sides of the bendable portion 1112 slightly protrude and deform outwards), and which can compensate for a lost stroke of the gripping body 110, so that the gripping body 110 can finally be locked to the locking structure.

Further, in order to reduce mutual interference between the gripping bodies 110 during the closing, in an embodiment, referring to FIG. 7, opposite ends of the gripping bodies 110 recessed inwards to form an avoidance structure 1110, so that an avoidance groove is formed between two opposite gripping bodies 110 through the recessed region. A width of the avoidance groove gradually increases in the first direction of the gripping body 110. An end of the avoidance groove adjacent to the gripping head 1111 is wider than another end of avoidance groove.

Further, referring to FIGS. 12 and 13, in an embodiment, a gripping body connecting structure 600 includes two connecting rods 610. The two connecting rods 610 each have an end connected to the distal end of the moving rod 200 and rotatable around a shaft 620 and another end connected to a cross shaft on the gripping head 1111 and also rotatable around the cross shaft. The connecting rods 610 assembly is Y-shaped, in order for effectively transferring push and pull forces caused by up-and-down motion of the moving rod 200 to the gripping head 1111 to realize control over the opening and closing of the gripping head 1111.

Further, referring to FIGS. 1 to 4, in an embodiment, the gripping body 110 includes a connecting portion 112. The connecting portion 112, the bendable portion 1112, and the gripping head 1111 are sequentially connected into an entirety. The first tearing portion 130 is connected between the connecting portion 112 and the separation base 120.

Referring to FIGS. 11 to 16, the connecting portion 112 has the engaging slot 1121 above. The engaging slot 1121 is configured to lock the gripping body 110 in the gripping state. The engaging slot 1121 may at least prevent motion of the gripping body 110 in the opening direction thereof, to ensure that the gripping body 110 is always in the gripping state.

The gripping body 110 forms a cylindrical structure. An end of the moving rod 200 extends into the cylindrical structure and is connected to the gripping body 110. The moving rod 200 is provided with an elastic piece 210. The elastic piece 210 is inclined towards the distal end of the gripping body 110 in a protruding direction thereof. When the moving rod 200 moves along the third stroke, the inclined elastic piece 210 may move towards the control handle 400 along an inner wall of the gripping body 110 to prevent the elastic piece 210 from being engaged with other parts of the gripping body 110. When the elastic piece 210 moves to a position corresponding to the engaging slot, the elastic piece 210 may be engaged into the engaging slot under an elastic force, to prevent the gripping state from being opened due to retraction of the moving rod 200 and the gripping body 110.

Further, in an embodiment, the moving rod 200 has an integrally formed structure, which has a retaining section 220 and a separation section 230. The retaining section 220 and the separation section 230 are connected into an entirety through a second tearing portion 240.

In order to assist the moving rod 200 in inner disengagement, referring to FIGS. 12 and 13, in an embodiment, the separation base 120 has a stopper 121. The stopper 121 is located on a movement path of the retaining section 220. When the moving rod 200 is located in the inner disengagement stroke, the stopper 121 prevents the retaining section 220 from continuously moving with the moving rod 200 and the separation section 230, to help separation of the retaining section 220 from the separation section 230.

In the embodiment shown in FIG. 14, the moving rod 200 has a slot 260 arranged in the axial direction thereof. The stopper 121 protrudes towards the moving rod 200 and extends into the slot 260 to abut against a wall of the slot 260 when the moving rod 200 moves along the inner disengagement stroke.

Further, referring to FIGS. 19 to 21, in an outer disengagement structure, at least one first tearing portion 130 is provided. An end of the gripping body 110 facing the separation base 120 has a concave region 1122. The first tearing portion 130 is arranged in the concave region 1122. The separation base 120 and the gripping body 110 are connected only through the tearing portion. In order to receive a uniform force, in an embodiment, the first tearing portion 130 is evenly distributed in the circumferential directions of the gripping body 110 and the separation base 120.

Referring to FIG. 16 and FIGS. 19 to 21, in an embodiment, the separation base 120 includes a cylindrical main body 122 and a suspension portion 124. A side wall of the main body 122 has a suspension cavity 123. The suspension portion 124 is placed in the suspension cavity 123. The suspension portion 124 is aligned with the first tearing portion 130.

Referring to FIGS. 12 to 16, the suspension portion 124 is provided with a follower 125. For example, the follower 125 is a follower shaft fixedly mounted on the suspension portion 124. The follower shaft traverses the suspension portion 124. The moving rod 200 may be further provided with a slide groove 250. The follower 125 is disposed at the bottom of the slide groove 250. As the moving rod 200 moves towards the control handle 400, when the moving rod 200 enters the outer disengagement stroke, the top of the slide groove 250 moves to the follower 125, thereby starting to drive the follower 125 and the suspension portion 124 to move towards the control handle 400 and then forcing the separation base 120 to be separated from the gripping body 110.

Referring to FIGS. 20 and 21, two sides of the suspension portion 124 are connected to the main body 122 through cantilevers 127 respectively. When the moving rod 200 moves along the outer disengagement stroke, the entire separation base 120 is supported by the rotating base 500 and the sleeve assembly 310, and cannot move towards control handle 400 alone. When the moving rod 200 pulls the suspension portion 124, the main body 122 of the separation base 120 remains stationary, while the cantilevers 127 of the suspension portion 124 are deformed under the pulling force of the moving rod 200. During the deformation of the suspension portion 124, the main body 122 of the separation base 120 forms a reverse support for the gripping body 110, and then materials of the cantilevers and the first tearing portion 130 are gradually elongated. As shown in FIG. 21, when a yield limit is reached, fracture occurs, and then the suspension portion 124 is separated from the gripping body 110. Thereafter, the separation base 120, the moving rod 200, and the transmission assembly 300 can be taken out from the surgical subject.

In order to prevent deformation of the suspension portion 124 in an undesired direction when the moving rod 200 pulls the suspension portion 124, referring to FIGS. 20 and 21, in an embodiment, the suspension cavity 123 has a guide groove 126 arranged in the axial direction of the separation base 120. The suspension portion 124 is disposed in the guide groove 126 to guide the suspension portion 124 to move into the guide groove 126. A guiding direction defined by the guide groove 126 is aligned with the first tearing portion 130, thereby making it easier for the suspension portion 124 to break at the first tearing portion 130.

Further, referring to FIGS. 1 and 2, in terms of the transmission assembly 300, the sleeve assembly 310 may generally include a spring support sleeve 311. The transmission member 320 (for example, a traction control wire) extends through the spring support sleeve 311. The moving rod 200 may be fixedly connected to the transmission member 320 through a reduction sleeve 321 or another structure. An adapter tube 312 fits over and is fixed to the spring support sleeve 311. The adapter tube 312 is rotationally connected to the rotating base 500. The gripper 100 is mounted on the rotating base 500, so that the entire gripper 100 can rotate relative to the transmission assembly 300 along with the rotating base 500.

## Claims

1. A manufacturing method for an integrated gripper, comprising:
providing a tubular base material;
forming a plurality of gripping heads (1111) facing each other by cutting the base material through a laser;
forming a plurality of columns of first shrinkage slits (1113) distributed in a first direction of the base material by cutting the base material through the laser, and each column of the first shrinkage slits (1113) corresponding to one of the gripping heads (1111) in the first direction;
forming at least two gripping arms (111) having first ends connected to each other and second ends separated from each other by cutting the base material in the first direction through the laser, each of the gripping arms (111) comprising one column of the first shrinkage slits (1113) and one of the gripping heads (1111); and
bending the gripping heads (1111) inwards to form a clamping jaw structure;
**characterized in that**
the first shrinkage slits (1113) all extending in a second direction of the base material.

2. A manufacturing method for an integrated gripper, comprising:
providing a sheet-like base material;
forming a plurality of gripping heads (1111) by cutting the base material through a laser;
forming a plurality of columns of first shrinkage slits (1113) distributed in a first direction of the base material by cutting the base material through the laser, and each column of the first shrinkage slits (1113) corresponding to one of the gripping heads (1111) in the first direction;
forming at least two gripping arms (111) having first ends connected to each other and second ends separated from each other by cutting the base material in the first direction through the laser, each of the gripping arms (111) comprising one column of the first shrinkage slits (1113) and one of the gripping heads (1111);
bending the base material around the first direction and fixing the bent base material to have a tubular shape; and
bending the gripping heads (1111) inwards to form a clamping jaw structure;
**characterized in that**
the first shrinkage slits (1113) all extending in a second direction of the base material.

3. The manufacturing method according to claim 1 or 2, further comprising:
forming a plurality of second shrinkage slits (1118) by cutting the base material in the second direction at side end surfaces on two sides of each column of the first shrinkage slits (1113),
wherein the second shrinkage slits (1118) on a same side are arranged in the first direction; two ends of each of the first shrinkage slits (1113) extend between two adjacent second shrinkage slits (1118) in the first direction; and an overlapping region between the first shrinkage slits (1113) and the second shrinkage slits (1118) forms a twist deformable section (1119), so that a bendable portion (1112) is capable of bending and twist deformation.

4. The manufacturing method according to claim 3, wherein a plurality of groups of first limiting blocks (1115) and second limiting blocks (1116) distributed in the first direction of the base material are formed by cutting for the second shrinkage slits (1118);
wherein the first limiting blocks (1115) and the second limiting blocks (1116) face each other, and the first limiting blocks (1115) and the second limiting blocks (1116) have first ends separated from each other and second ends connected into an entirety; gaps (1117) arranged in the first direction are respectively defined between the first limiting blocks (1115) and the second limiting blocks (1116); and the second shrinkage slits (1118) are in communication with the gaps (1117).

5. The manufacturing method according to claim 4, wherein each of the second shrinkage slits (1118) has a clearance in the first direction.

6. The manufacturing method according to claim 4 or 5, wherein each of the first limiting blocks (1115) and the second limiting blocks (1116) has a hook structure.

7. The manufacturing method according to any one of claims 1 to 6, comprising:
forming a notch extending in the first direction of the base material by cutting opposed ends of the gripping arms (111) through the laser, to form an avoidance structure (1110).

8. The manufacturing method according to any one of claims 1 to 7, comprising:
forming an engaging slot (1121) by cutting the first ends connected to each other of the gripping arms (111) through the laser, the engaging slot (1121) being configured to lock the gripping arm (111).

9. The manufacturing method according to claim 8, wherein at least two engaging slots (1121) are provided and are distributed in the second direction of the base material.

10. The manufacturing method according to any one of claims 1 to 9, comprising:
forming a separation base (120) by cutting an end of the base material facing away from the gripping arms (111) through the laser, the separation base (120) and the gripping arms (111) being connected into an entirety through a first tearing portion (130).

11. The manufacturing method according to claim 10, wherein at least two first tearing portions (130) are provided and are distributed in the second direction of the base material.

12. The manufacturing method according to claim 10 or 11, comprising:
forming separation grooves (1122) recessed towards the gripping arms (111) by cutting two sides of the first tearing portion (130) through the laser, the first tearing portion (130) being located at a recess.

13. The manufacturing method according to any one of claims 10 to 12, comprising:
forming a suspended suspension portion (121) and cantilevers (127) located on two sides of the suspension portion (121) by cutting the separation base (120) through the laser, the suspension portion (121) being aligned with the first tearing portion (130);
and optionally, the manufacturing method further comprises:
forming a guide groove (126) arranged in a first direction of the separation base (120) by cutting a side of the suspension portion (121) away from the first tearing portion (130) through the laser, the suspension portion (121) being disposed in the guide groove (126).

14. The manufacturing method according to any one of claims 10 to 13, comprising:
forming a stopper (121) by cutting the separation base (120) through the laser; and
pressing and deforming the stopper (121) towards an interior of the separation base (120), to cause the stopper (121) to protrude towards the interior of the separation base (120).

15. An integrated gripper, manufactured by the manufacturing method according to any one of claims 1 to 14.

## Patentansprüche

1. Ein Herstellungsverfahren für einen integrierten Greifer, das Folgendes beinhaltet:
Bereitstellen eines röhrenförmigen Basismaterials;
Bilden einer Vielzahl von Greifköpfen (1111), die einander zugewandt sind, durch Schneiden des Basismaterials durch einen Laser;
Bilden einer Vielzahl von Spalten von ersten Schrumpfschlitzen (1113), die in einer ersten Richtung des Basismaterials verteilt sind, durch Schneiden des Basismaterials durch den Laser, und wobei jede Spalte der ersten Schrumpfschlitze (1113) einem der Greifköpfe (1111) in der ersten Richtung entspricht;
Bilden von mindestens zwei Greifarmen (111) mit ersten Enden, die miteinander verbunden sind, und zweiten Enden, die voneinander getrennt sind, durch Schneiden des Basismaterials in der ersten Richtung durch den Laser, wobei jeder der Greifarme (111) eine Spalte der ersten Schrumpfschlitze (1113) und einen der Greifköpfe (1111) beinhaltet; und
Biegen der Greifköpfe (1111) nach innen, um eine Klemmbackenstruktur zu bilden;
**dadurch gekennzeichnet, dass**
sich die ersten Schrumpfschlitze (1113) alle in einer zweiten Richtung des Basismaterials erstrecken.

2. Ein Herstellungsverfahren für einen integrierten Greifer, das Folgendes beinhaltet:
Bereitstellen eines blattartigen Basismaterials;
Bilden einer Vielzahl von Greifköpfen (1111) durch Schneiden des Basismaterials durch einen Laser;
Bilden einer Vielzahl von Spalten von ersten Schrumpfschlitzen (1113), die in einer ersten Richtung des Basismaterials verteilt sind, durch Schneiden des Basismaterials durch den Laser, und wobei jede Spalte der ersten Schrumpfschlitze (1113) einem der Greifköpfe (1111) in der ersten Richtung entspricht;
Bilden von mindestens zwei Greifarmen (111) mit ersten Enden, die miteinander verbunden sind, und zweiten Enden, die voneinander getrennt sind, durch Schneiden des Basismaterials in der ersten Richtung durch den Laser, wobei jeder der Greifarme (111) eine Spalte der ersten Schrumpfschlitze (1113) und einen der Greifköpfe (1111) beinhaltet;
Biegen des Basismaterials um die erste Richtung und Fixieren des gebogenen Basismaterials, sodass es eine Röhrenform aufweist; und
Biegen der Greifköpfe (1111) nach innen, um eine Klemmbackenstruktur zu bilden;
**dadurch gekennzeichnet, dass**
sich die ersten Schrumpfschlitze (1113) alle in einer zweiten Richtung des Basismaterials erstrecken.

3. Herstellungsverfahren gemäß Anspruch 1 oder 2, das ferner Folgendes beinhaltet:
Bilden einer Vielzahl von zweiten Schrumpfschlitzen (1118) durch Schneiden des Basismaterials in der zweiten Richtung an Seitenendflächen auf zwei Seiten jeder Spalte der ersten Schrumpfschlitze (1113),
wobei die zweiten Schrumpfschlitze (1118) auf einer gleichen Seite in der ersten Richtung angeordnet sind; sich zwei Enden jedes der ersten Schrumpfschlitze (1113) zwischen zwei benachbarten zweiten Schrumpfschlitzen (1118) in der ersten Richtung erstrecken; und ein Überlappungsbereich zwischen den ersten Schrumpfschlitzen (1113) und den zweiten Schrumpfschlitzen (1118) einen verdrehverformbaren Abschnitt (1119) bildet, sodass ein biegbarer Abschnitt (1112) in der Lage ist, sich zu biegen und sich zu verdrehverformen.

4. Herstellungsverfahren gemäß Anspruch 3, wobei eine Vielzahl von Gruppen von ersten Begrenzungsblöcken (1115) und zweiten Begrenzungsblöcken (1116), die in der ersten Richtung des Basismaterials verteilt sind, durch Schneiden für die zweiten Schrumpfschlitze (1118) gebildet werden;
wobei die ersten Begrenzungsblöcke (1115) und die zweiten Begrenzungsblöcke (1116) einander zugewandt sind und die ersten Begrenzungsblöcke (1115) und die zweiten Begrenzungsblöcke (1116) erste Enden, die voneinander getrennt sind, und zweite Enden, die in einer Gesamtheit verbunden sind, aufweisen; Lücken (1117), die in der ersten Richtung angeordnet sind, jeweils zwischen den ersten Begrenzungsblöcken (1115) und den zweiten Begrenzungsblöcken (1116) definiert sind; und die zweiten Schrumpfschlitze (1118) mit den Lücken (1117) in Verbindung stehen.

5. Herstellungsverfahren gemäß Anspruch 4, wobei jeder der zweiten Schrumpfschlitze (1118) einen Zwischenraum in der ersten Richtung aufweist.

6. Herstellungsverfahren gemäß Anspruch 4 oder 5, wobei jeder der ersten Begrenzungsblöcke (1115) und der zweiten Begrenzungsblöcke (1116) eine Hakenstruktur aufweist.

7. Herstellungsverfahren gemäß einem der Ansprüche 1 bis 6, das Folgendes beinhaltet:
Bilden einer Kerbe, die sich in der ersten Richtung des Basismaterials erstreckt, durch Schneiden gegenüberliegender Enden der Greifarme (111) durch den Laser, um eine Ausweichstruktur (1110) zu bilden.

8. Herstellungsverfahren gemäß einem der Ansprüche 1 bis 7, das Folgendes beinhaltet:
Bilden eines Eingriffsschlitzes (1121) durch Schneiden der ersten Enden, die miteinander verbunden sind, der Greifarme (111) durch den Laser, wobei der Eingriffsschlitz (1121) dazu konfiguriert ist, den Greifarm (111) zu verriegeln.

9. Herstellungsverfahren gemäß Anspruch 8, wobei mindestens zwei Eingriffsschlitze (1121) bereitgestellt sind und in der zweiten Richtung des Basismaterials verteilt sind.

10. Herstellungsverfahren gemäß einem der Ansprüche 1 bis 9, das Folgendes beinhaltet:
Bilden einer Trennbasis (120) durch Schneiden eines Endes des Basismaterials, das von den Greifarmen (111) abgewandt ist, durch den Laser, wobei die Trennbasis (120) und die Greifarme (111) in einer Gesamtheit durch einen ersten Reißabschnitt (130) verbunden sind.

11. Herstellungsverfahren gemäß Anspruch 10, wobei mindestens zwei erste Reißabschnitte (130) bereitgestellt sind und in der zweiten Richtung des Basismaterials verteilt sind.

12. Herstellungsverfahren gemäß Anspruch 10 oder 11, das Folgendes beinhaltet:
Bilden von Trennnuten (1122), die zu den Greifarmen (111) hin ausgespart sind, durch Schneiden zweier Seiten des ersten Reißabschnitts (130) durch den Laser, wobei sich der erste Reißabschnitt (130) an einer Aussparung befindet.

13. Herstellungsverfahren gemäß einem der Ansprüche 10 bis 12, das Folgendes beinhaltet:
Bilden eines aufgehängten Aufhängungsabschnitts (121) und von Auslegern (127), die sich auf zwei Seiten des Aufhängungsabschnitts (121) befinden, durch Schneiden der Trennbasis (120) durch den Laser, wobei der Aufhängungsabschnitt (121) auf den ersten Reißabschnitt (130) ausgerichtet ist;
und optional das Herstellungsverfahren ferner Folgendes beinhaltet:
Bilden einer Führungsnut (126), die in einer ersten Richtung der Trennbasis (120) angeordnet ist, durch Schneiden einer Seite des Aufhängungsabschnitts (121) von dem ersten Reißabschnitt (130) weg durch den Laser, wobei der Aufhängungsabschnitt (121) in der Führungsnut (126) eingerichtet ist.

14. Herstellungsverfahren gemäß einem der Ansprüche 10 bis 13, das Folgendes beinhaltet:
Bilden eines Anschlags (121) durch Schneiden der Trennbasis (120) durch den Laser;
und
Drücken und Verformen des Anschlags (121) in Richtung eines Inneren der Trennbasis (120), um zu bewirken, dass der Anschlag (121) in Richtung des Inneren der Trennbasis (120) vorsteht.

15. Ein integrierter Greifer, der durch das Herstellungsverfahren gemäß einem der Ansprüche 1 bis 14 hergestellt wird.

## Revendications

1. Un procédé de fabrication pour un dispositif de préhension intégré, comprenant :
la fourniture d'un matériau de base tubulaire ;
la formation d'une pluralité de têtes de préhension (1111) se faisant face en découpant le matériau de base par l'intermédiaire d'un laser ;
la formation d'une pluralité de colonnes de premières fentes de rétrécissement (1113) réparties dans une première direction du matériau de base en découpant le matériau de base par l'intermédiaire du laser, et chaque colonne des premières fentes de rétrécissement (1113) correspondant à l'une des têtes de préhension (1111) dans la première direction ;
la formation d'au moins deux bras de préhension (111) ayant des premières extrémités reliées l'une à l'autre et des deuxièmes extrémités séparées l'une de l'autre en découpant le matériau de base dans la première direction par l'intermédiaire du laser, chacun des bras de préhension (111) comprenant une colonne des premières fentes de rétrécissement (1113) et l'une des têtes de préhension (1111) ; et
le fléchissement des têtes de préhension (1111) vers l'intérieur pour former une structure de mâchoire de serrage ;
**caractérisé en ce que**
les premières fentes de rétrécissement (1113) s'étendent toutes dans une deuxième direction du matériau de base.

2. Un procédé de fabrication pour un dispositif de préhension intégré, comprenant :
la fourniture d'un matériau de base semblable à une feuille ;
la formation d'une pluralité de têtes de préhension (1111) en découpant le matériau de base par l'intermédiaire d'un laser ;
la formation d'une pluralité de colonnes de premières fentes de rétrécissement (1113) réparties dans une première direction du matériau de base en découpant le matériau de base par l'intermédiaire du laser, et chaque colonne des premières fentes de rétrécissement (1113) correspondant à l'une des têtes de préhension (1111) dans la première direction ;
la formation d'au moins deux bras de préhension (111) ayant des premières extrémités reliées l'une à l'autre et des deuxièmes extrémités séparées l'une de l'autre en découpant le matériau de base dans la première direction par l'intermédiaire du laser, chacun des bras de préhension (111) comprenant une colonne des premières fentes de rétrécissement (1113) et l'une des têtes de préhension (1111) ;
le fléchissement du matériau de base autour de la première direction et la fixation du matériau de base fléchi pour qu'il ait une forme tubulaire ; et
le fléchissement des têtes de préhension (1111) vers l'intérieur pour former une structure de mâchoire de serrage ;
**caractérisé en ce que**
les premières fentes de rétrécissement (1113) s'étendent toutes dans une deuxième direction du matériau de base.

3. Le procédé de fabrication selon la revendication 1 ou la revendication 2, comprenant en outre :
la formation d'une pluralité de deuxièmes fentes de rétrécissement (1118) en découpant le matériau de base dans la deuxième direction au niveau de surfaces d'extrémité latérales sur deux côtés de chaque colonne des premières fentes de rétrécissement (1113),
dans lequel les deuxièmes fentes de rétrécissement (1118) sur un même côté sont agencées dans la première direction ; deux extrémités de chacune des premières fentes de rétrécissement (1113) s'étendent entre deux deuxièmes fentes de rétrécissement (1118) adjacentes dans la première direction ; et une région de chevauchement entre les premières fentes de rétrécissement (1113) et les deuxièmes fentes de rétrécissement (1118) forme une section déformable en torsion (1119), de sorte qu'une partie fléchissable (1112) est apte à fléchir et à se déformer en torsion.

4. Le procédé de fabrication selon la revendication 3, dans lequel une pluralité de groupes de premiers blocs de limitation (1115) et de deuxièmes blocs de limitation (1116) répartis dans la première direction du matériau de base sont formés en découpant pour les deuxièmes fentes de rétrécissement (1118) ;
dans lequel les premiers blocs de limitation (1115) et les deuxièmes blocs de limitation (1116) se font face, et les premiers blocs de limitation (1115) et les deuxièmes blocs de limitation (1116) ont des premières extrémités séparées l'une de l'autre et des deuxièmes extrémités reliées dans leur intégralité ; des espaces (1117) agencés dans la première direction sont respectivement définis entre les premiers blocs de limitation (1115) et les deuxièmes blocs de limitation (1116) ; et les deuxièmes fentes de rétrécissement (1118) sont en communication avec les espaces (1117).

5. Le procédé de fabrication selon la revendication 4, dans lequel chacune des deuxièmes fentes de rétrécissement (1118) a un espace libre dans la première direction.

6. Le procédé de fabrication selon la revendication 4 ou la revendication 5, dans lequel chacun des premiers blocs de limitation (1115) et des deuxièmes blocs de limitation (1116) a une structure en crochet.

7. Le procédé de fabrication selon l'une quelconque des revendications 1 à 6, comprenant :
la formation d'une encoche s'étendant dans la première direction du matériau de base en découpant des extrémités opposées des bras de préhension (111) par l'intermédiaire du laser, pour former une structure d'évitement (1110).

8. Le procédé de fabrication selon l'une quelconque des revendications 1 à 7, comprenant :
la formation d'une entaille d'engagement (1121) en découpant les premières extrémités reliées l'une à l'autre des bras de préhension (111) par l'intermédiaire du laser, l'entaille d'engagement (1121) étant configurée pour verrouiller le bras de préhension (111).

9. Le procédé de fabrication selon la revendication 8, dans lequel au moins deux entailles d'engagement (1121) sont prévues et sont réparties dans la deuxième direction du matériau de base.

10. Le procédé de fabrication selon l'une quelconque des revendications 1 à 9, comprenant :
la formation d'une base de séparation (120) en découpant une extrémité du matériau de base tournant le dos aux bras de préhension (111) par l'intermédiaire du laser, la base de séparation (120) et les bras de préhension (111) étant reliés dans leur intégralité par l'intermédiaire d'une première partie de déchirement (130).

11. Le procédé de fabrication selon la revendication 10, dans lequel au moins deux premières parties de déchirement (130) sont prévues et sont réparties dans la deuxième direction du matériau de base.

12. Le procédé de fabrication selon la revendication 10 ou la revendication 11, comprenant :
la formation de rainures de séparation (1122) évidées vers les bras de préhension (111) en découpant deux côtés de la première partie de déchirement (130) par l'intermédiaire du laser, la première partie de déchirement (130) étant située au niveau d'un évidement.

13. Le procédé de fabrication selon l'une quelconque des revendications 10 à 12, comprenant :
la formation d'une partie de suspension (121) suspendue et de porte-à-faux (127) situés sur deux côtés de la partie de suspension (121) en découpant la base de séparation (120) par l'intermédiaire du laser, la partie de suspension (121) étant alignée avec la première partie de déchirement (130) ;
et facultativement, le procédé de fabrication comprend en outre :
la formation d'une rainure de guidage (126) agencée dans une première direction de la base de séparation (120) en éloignant un côté de la partie de suspension (121) de la première partie de déchirement (130) par découpe par l'intermédiaire du laser, la partie de suspension (121) étant disposée dans la rainure de guidage (126).

14. Le procédé de fabrication selon l'une quelconque des revendications 10 à 13, comprenant :
la formation d'une butée (121) en découpant la base de séparation (120) par l'intermédiaire du laser ; et
la pression et la déformation de la butée (121) vers un intérieur de la base de séparation (120), pour amener la butée (121) à faire saillie vers l'intérieur de la base de séparation (120).

15. Un dispositif de préhension intégré, fabriqué par le procédé de fabrication selon l'une quelconque des revendications 1 à 14.
